(19) Europäisches Patentamt
European Patent Office
Office européen des brevets

(11) EP 1 688 140 A1

(12) EUROPEAN PATENT APPLICATION
published in accordance with Art. 158(3) EPC

(43) Date of publication:
**09.08.2006 Bulletin 2006/32**

(21) Application number: **04793368.4**

(22) Date of filing: **28.10.2004**

(51) Int Cl.:
***A61K 31/352*** *(2006.01)* ***A61P 3/06*** *(2006.01)*
***A23L 1/30*** *(2006.01)*

(86) International application number:
**PCT/JP2004/016448**

(87) International publication number:
**WO 2005/046706 (26.05.2005 Gazette 2005/21)**

(84) Designated Contracting States:
**AT BE BG CH CY CZ DE DK EE ES FI FR GB GR HU IE IT LI LU MC NL PL PT RO SE SI SK TR**

(30) Priority: **17.11.2003 JP 2003386674**
**17.11.2003 JP 2003386675**
**10.05.2004 JP 2004140142**
**11.06.2004 JP 2004174396**

(71) Applicant: **Toyo Shinyaku Co., Ltd.**
**Fukuoka-shi, Fukuoka 812-0011 (JP)**

(72) Inventors:
- **TAKAGAKI, Kinya,**
**Toyo Shinyaku Co., Ltd.**
**Fukuoka-shi,**
**Fukuoka 8120011 (JP)**
- **MORI, Sadao,**
**Toyo Shinyaku Co., Ltd.**
**Fukuoka-shi,**
**Fukuoka 8120011 (JP)**
- **TSUBATA, Masahito,**
**Toyo Shinyaku Co., Ltd.**
**Fukuoka-shi,**
**Fukuoka 8120011 (JP)**

(74) Representative: **Arth, Hans-Lothar**
**Arth, Bucher & Kollegen,**
**Am Klopferspitz 19**
**82152 Martinsried (DE)**

# (54) LIPOMETABOLISM IMPROVER CONTAINING PINE BARK EXTRACT

(57) As a lipid metabolism improving agent for allowing lipids in the body to be metabolized efficiently, a lipid metabolism improving agent containing a pine bark extract as an active component is provided. The lipid metabolism improving agent of the present invention has abilities to promote cholesterol excretion, inhibit lipid absorption, reduce body fat, and inhibit fat accumulation, and the like.

EP 1 688 140 A1

## Description

Technical Field

The present invention relates to a lipid metabolism improving agent containing a pine bark extract as an active component.

Background Art

Hyperlipemia is one of symptoms that have been receiving attention as the causes of arteriosclerosis or cerebral stroke. In order to prevent or treat hyperlipemia, various methods or drugs have been studied. For example, Japanese Patent No. 3393304 discloses a hyperlipemia preventing or treating agent containing a peptide that is obtained by hydrolyzing a corn protein and that has an angiotensin converting enzyme inhibiting activity.

In addition to this, compounds having an ability to inhibit cholesterol biosynthesis in the liver, such as mevalotin (registered trademark) or ML-236B derivatives described in Japanese Patent Publication No. 61-13699, have been known. Furthermore, substances having an ability of excreting cholesterol from the body by inhibiting cholesterol absorption, such as chitosan and derivatives thereof, or materials containing such a substance have been known (Japanese Patent No. 3108675 and Japanese Patent Publication No. 8-19001). Administration of these substances or materials has been practiced to reduce the amount of cholesterol in the body, thereby improving lipids in blood.

However, since the mechanism of action of mevalotin and the ML-236B derivatives mentioned above is inhibition of cholesterol biosynthesis, there is a possibility for even biosynthesis of the cholesterol that is necessary in the body to be inhibited. Thus, the true state is that mevalotin and the ML-236B derivatives have been used mainly for the treatment of hyperlipemia, and are not suited for the prevention of hyperlipemia. Chitosan and the derivatives thereof mentioned above have an ability of excreting cholesterol from the body, and thus, these substances can reduce the cholesterol in the body. Furthermore, these substances can inhibit internal absorption of cholesterol ingested in the diet. Thus, chitosan is most effective in treating and preventing hyperlipemia, and has received growing attention in recent years. However, chitosan involves problems such as food allergy, so that care should be taken in use. Although chitosan is used in combination with a protein hydrolyzate in the description of Japanese Patent No. 3108675 mentioned above, the effect is still less than satisfactory.

Moreover, in order to prevent hyperlipemia, attempts have been made to promote absorption of cholesterol into the liver by reducing low density lipoproteins (LDL) and increasing high density lipoproteins (HDL) in blood.

For such a purpose, for example, Japanese Laid-Open Patent Publication No. 2002-223727 discloses a functional food product containing hyaluronic acid and fucoidan, Japanese Laid-Open Patent Publication (tokuhyo) No. 11-507910 discloses a composition containing an immunogenic epitope of cholesterol ester transfer protein, and Japanese Laid-Open Patent Publication (tokuhyo) No. 2000-505308 discloses a nutritional composition containing a soy protein isolate and a soy fiber.

Japanese Laid-Open Patent Publication (tokuhyo) No. 11-515025 discloses that HDL is increased by administering an acyl-CoA cholesterol O-acyl transferase (ACAT) inhibitor and a HMG-CoA reductase inhibitor. Furthermore, attempts have been made to treat or prevent hyperlipemia by promoting degradation of cholesterol in the liver or promoting cholesterol excretion together with bile acid to reduce lipids such as cholesterol in the body.

However, in these technologies, a heavy burden due to lipid metabolism is imposed on the liver, resulting in a decrease in liver function. For example, application of a load due to hepatitis virus infection, alcohol ingestion, medication, and the like causes an imbalance between metabolism and absorption of cholesterol in the liver, resulting in increased accumulation of lipids in the liver, and thus fatty liver may develop as a complication. If a state in which lipids are accumulated in the liver continues, then the liver tissue may undergo necrosis, and when the cells infiltrate into the necrotic tissue portion, fibrosis of the liver occurs (i.e., cirrhosis develops). Thus, lipid metabolism in the liver is an important function in maintaining good health in the body, and accumulation of fats in the liver may lead to serious diseases and disorders. Moreover, accumulation of fats in the body causes obesity, and resulting adult diseases, e.g., hypertension, fatty liver, hepatitis, and cirrhosis, also have become a social issue.

Accordingly, attempts have been made to prevent accumulation of fats in the body by inhibiting fat absorption (Japanese Laid-Open Patent Publication No. 8-259461 and Japanese Laid-Open Patent Publication No. 2001-226274).

However, a sufficient effect has not been obtained, and in particular, there is a problem in that lipid absorption cannot be inhibited sufficiently even though a lipase inhibiting ability is provided.

Disclosure of Invention

The inventors of the present invention conducted in-depth research on components for improving lipid metabolism described above. As a result, it was found that a pine bark extract has a superior effect of improving lipid metabolism, and thus the present invention was achieved.

The present invention provides a lipid metabolism improving agent. This lipid metabolism improving agent comprises a pine bark extract as an active component.

In a preferred embodiment, the lipid metabolism improving agent is a lipid absorption inhibiting agent.

In a preferred embodiment, the lipid metabolism improving agent is a cholesterol excretion promoting agent.

In a preferred embodiment, the lipid metabolism improving agent is a body fat reducing agent.

In a preferred embodiment, the lipid metabolism improving agent is a fat accumulation inhibitory agent, wherein the fat is visceral fat or subcutaneous fat.

Brief Description of Drawings

Fig. 1 is a graph showing that a lipid metabolism improving agent (a lipid absorption inhibiting agent containing a pine bark extract) of the present invention in Example 1 suppresses an increase in neutral fat in blood due to administration of cottonseed oil.

Best Mode for Carrying Out the Invention

A lipid metabolism improving agent of the present invention contains a pine bark extract as an active component. The lipid metabolism improving agent may further contain, in addition to the pine bark extract, a functional component, a nutrition, and an additive, if necessary. Hereinafter, the components to be contained in the lipid metabolism improving agent of the present invention and the lipid metabolism improving agent of the present invention will be described. It should be noted that the following description should not be construed as limiting the present invention, and it will be apparent to those skilled in the art that various alternations can be made within the scope of the present invention.

(I) Pine bark extract

As a pine bark serving as a raw material of the pine bark extract, the bark of plant belonging to Pinales, such as French maritime pine (Pinus Martima), Larix Leptolepis, Pinus thunbergii, Pinus densiflora, Pinus parviflora, Pinus pentaphylla, Pinus koraiensis, Pinus pumila, Pinus luchuensis, utsukushimatsu (Pinus densiflora form. umbraculifera), Pinus palustris, Pinus bungeana, and Anneda in Quebec, Canada, are preferably used. Among these, French maritime pine (Pinus Martima) bark is preferably used.

French maritime pine refers to maritime pines that grow in a part of the Atlantic coastal area in southern France. The bark of this French maritime pine contains proanthocyanidins, organic acids, and other bioactive substances, and the like.

The pine bark extract used in the present invention is obtained by extracting the bark of the above-described pines using water or an organic solvent. When water is used, warm water or hot water can be employed. When an organic solvent is used, a solvent that is acceptable for production of food products or pharmaceuticals can be employed. Examples of such an organic solvent include methanol, ethanol, 1-propanol, 2-propanol, 1-butanol, 2-butanol, acetone, hexane, cyclohexane, propylene glycol, aqueous ethanol, aqueous propylene glycol, methyl ethyl ketone, glycerin, methyl acetate, ethyl acetate, diethyl ether, dichloromethane, edible oils or fats, 1,1,1,2-tetrafluoroethane, and 1,1,2-trichloroethene. These water and organic solvents may be used alone or in combination of two or more. In particular, water, ethanol, aqueous ethanol, and aqueous propylene glycol are preferably used. In view of the safety when used in food products or pharmaceuticals, water, ethanol, and aqueous ethanol are more preferable, and are more preferably warmed before extraction.

There is no particular limitation on the method for extraction from pine bark, and heat extraction or supercritical fluid extraction can be employed, for example.

Supercritical fluid extraction is a method for performing extraction using a supercritical fluid. A supercritical fluid is in a state that is above the liquid-vapor critical point in the phase diagram showing critical temperature and critical pressure. Examples of compounds that can be employed as a supercritical fluid include carbon dioxide, ethylene, propane, and nitrous oxide (laughter gas). Carbon dioxide is preferably used.

Supercritical fluid extraction includes an extraction step in which a target component is extracted with a supercritical fluid and a separation step in which the target component is separated from the supercritical fluid. In the separation step, any separation process can be employed, examples of which include a separation based on a change in pressure, a separation based on a change in temperature, and a separation based on an adsorbent or absorbent.

Moreover, it is also possible to perform supercritical fluid extraction in which an entrainer is added. In this method, for example, about 2 to 20 W/V% of ethanol, propanol, n-hexane, acetone, toluene, or another aliphatic lower alcohol, aliphatic hydrocarbon, aromatic hydrocarbon, or ketone is added to the above-described fluid capable of forming a supercritical fluid, and the resultant fluid is turned to a supercritical fluid state and used to extract a target substance. With this method, the solubility of a target substance to be extracted, such as proanthocyanidins and catechins, in the extracting solvent can be dramatically increased, or the selectivity of separation can be enhanced. Thus a pine bark extract can be obtained efficiently.

Since supercritical fluid extraction can be performed at a relatively low temperature, it has the following advantages: it is applicable for extracting substances that deteriorate or decompose at high temperatures; the extracting fluid does not remain; and the extracting fluid can be recovered and recycled, so that a step of removing the extracting fluid and the like can be omitted, and thus, the process can be simplified.

Furthermore, methods other than those mentioned above can be employed for extraction from pine bark, examples of which include a batch method using liquid carbon dioxide, a reflux method using liquid carbon dioxide, and a reflux method using supercritical carbon dioxide.

It is also possible to employ a combination of a plurality of extraction processes to perform extraction from pine bark. By combining a plurality of extraction processes, pine bark extracts with various components can be obtained.

In view of the safety, it is preferable to purify the pine bark extract obtained by performing extraction as described above by ultrafiltration or by a column chromatography method or a batch method using an adsorptive carrier (e.g., DIAION HP-20, Sephadex-LH20, or chitin).

The pine bark extract that is used in the lipid metabolism improving agent of the present invention is specifically prepared using the following method. However, this method is merely an example and the present invention is not limited to this method.

First, 1 kg of the bark of French maritime pine is immersed in 3 L of a saturated aqueous solution of sodium chloride, and extraction is performed for 30 minutes at 100°C to obtain an extract liquid (extraction step). Then, the extract liquid is filtrated, and the resultant insoluble material is washed with 500 mL of a saturated solution of sodium chloride to obtain a washed liquid (washing step). The extract liquid and the washed liquid are combined to obtain a crude extract liquid of pine bark.

Next, 250 mL of ethyl acetate are added to this crude extract liquid, mixed, and separated to obtain an ethyl acetate layer. This process is repeated five times, and the obtained ethyl acetate layers are combined. The resultant ethyl acetate extract is added directly to 200 g of anhydrous sodium sulfate for drying. Then, this ethyl acetate extract is filtrated, and the filtrated extract is concentrated under a reduced pressure to a volume of 1/5 of the original filtrated extract. The concentrated ethyl acetate extract is poured into 2 L of chloroform and stirred, and the resultant precipitate is recovered by filtration. Subsequently, this precipitate is dissolved in 100 mL of ethyl acetate, and then the resultant solution is added to 1 L of chloroform to form a precipitate. This process is repeated twice for washing. With this method, for example, about 5 g of a pine bark extract containing at least 20 wt% of proanthocyanidins having a degree of polymerization of 2 to 4 and at least 5 wt% of catechins can be obtained. Here, the content of a particular component in the extract is a value based on the dry weight of the extract. This applies to the following description.

The pine bark extract used in the present invention contains proanthocyanidins as one of main active components. Proanthocyanidins refer to a group of compounds that are condensation products having flavan-3-ol and/or flavan-3,4-diol as a constituent unit and having a degree of polymerization of 2 or more. Proanthocyanidins are potent antioxidants produced by plants, and contained concentratedly in plant leaves, bark, or skin or seeds of fruits. Proanthocyanidins cannot be produced in the human body.

When a pine bark extract containing proanthocyanidins is ingested, a superior effect of improving lipid metabolism can be provided. A pine bark extract contains condensation products having a degree of polymerization of 2 or more as the proanthocyanidins, and further contains catechins and the like. In particular, proanthocyanidins containing a large amount of condensation products having a lower degree of polymerization are preferably used. As such condensation products having a lower degree of polymerization, condensation products having a degree of polymerization of 2 to 30 (dimer to 30-mer) are preferable, condensation products having a degree of polymerization of 2 to 10 (dimer to decamer) are more preferable, and condensation products having a degree of polymerization of 2 to 4 (dimer to tetramer) are even more preferable. Proanthocyanidins that are condensation products having a degree of polymerization of 2 to 4 (dimer to tetramer) are particularly easily absorbed into the body, and therefore it seems that, in addition to the ability to promote cholesterol excretion, an ability to promote degradation of neutral fat and other abilities can be provided more efficiently. In the present specification, the above-mentioned condensation products having a degree of polymerization of 2 to 4 are referred to as oligomeric proanthocyanidins (hereinafter, abbreviated as "OPCs").

Proanthocyanidins having a degree of polymerization of 5 or more are considered to have abilities to promote cholesterol excretion and inhibition of neutral fat absorption. As the pine bark extract, an extract containing condensation products having a degree of polymerization of 2 to 4 (dimer to tetramer; i.e., OPCs) in a ratio of 15 wt% or more, preferably 20 wt% or more, more preferably 30 wt% or more, and containing proanthocyanidins having a degree of polymerization of 5 or more in a ratio of 10 wt% or more, preferably 15 wt% or more, is preferable. As described above, since the pine bark extract contains OPCs and proanthocyanidins having a degree of polymerization of 5 or more, it seems that the cholesterol excretion promoting ability, the fat absorption inhibiting ability, the body fat reducing ability, and the like can be provided. As a synergistic effect of such abilities, lipid metabolism in the body can be improved.

The pine bark extract described above may further contain catechins, and the catechins can be contained in a ratio of preferably 5 wt% or more, more preferably 10 wt% or more. Catechins may be extracted together with proanthocyanidins (OPCs) using the above-described extraction methods. The term "catechins" is a general term referring to polyhydroxyflavan-3-ols. As the catechins, for example, (+)-catechin that is called catechin in a narrow sense, (-)-epicatechin, (+)-gallocatechin, (-)-epigallocatechin, epigallocatechin gallate, epicatechin gallate, and afzelechin are known. From the pine bark extract described above, gallocatechin, afzelechin, and 3-galloyl derivatives of (+)-catechin or gallocatechin are isolated in addition to (+)-catechin. Catechins are known to have a cancer inhibiting ability, an arteriosclerosis preventing ability, a blood pressure elevation inhibiting ability, a platelet aggregation inhibiting ability, an antiallergic ability, an antiviral ability, an antibacterial ability, a dental caries preventing ability, a halitosis preventing ability, an intestinal flora normalization ability, an active oxygen or free radical eliminating ability, an antioxidation ability, and the like. Moreover, catechins are known to have an antidiabetic ability to inhibit an elevation of blood glucose. Catechins alone have poor water solubility and exhibit low bioactivity, but the water solubility of catechins is increased in the presence of OPCs, and the activities of catechins are activated in the presence of OPCs. Therefore, catechins work effectively when ingested together with OPCs.

It is preferable that catechins are contained in the above-described pine bark extract in a ratio of 5 wt% or more. More preferably, it is preferable that catechins are contained in a ratio of 5 wt% or more in a pine bark extract containing at least 20 wt% of OPCs and at least 10 wt% of proanthocyanidins having a degree of polymerization of 5 or more. For example, when the catechin content in a pine bark extract is less than 5 wt%, it is possible to add catechins so that the catechin content becomes at least 5 wt%. It is most preferable to use a pine bark extract containing at least 5 wt% of catechins, at least 20 wt% of OPCs, and at least 10 wt% of proanthocyanidins having a degree of polymerization of 5 or more.

(II) Functional component

Examples of the aforementioned functional component that may be contained in the lipid metabolism improving agent of the present invention include ascorbic acid and derivatives thereof, mucopolysaccharides, amino sugars, flavonoids, vitamins other than ascorbic acid, and water-soluble dietary fibers.

Ascorbic acid or a derivative thereof can allow proanthocyanidins, in particular, OPCs, in the pine bark extract to exert their effects more efficiently. As the derivative of ascorbic acid, derivatives of ascorbic acid that are usually used as food additives can be employed. Examples of the derivative of ascorbic acid include ascorbyl glycoside, sodium ascorbate, and magnesium ascorbate. Moreover, natural materials that contain ascorbic acid abundantly (e.g., natural materials derived from fruits such as lemon, orange, and acelora or natural materials derived from vegetables such as broccoli, Brussels sprouts, pimento, Brassica campestris, and cauliflower) also can be utilized.

It is known that when ascorbic acid is ingested together with the above-described OPCs, the absorptivity and the persistence of bioactivity of ascorbic acid are increased. In the present invention, ascorbic acid or a derivative thereof may be contained in order to protect blood vessels, especially in order to enhance the flexibility and strength of blood vessels and to decrease cholesterol in blood. In particular, ascorbic acid is known to have an ability of promoting synthesis of collagen that is a structural protein not only of blood vessels but also of every tissue, an ability of reducing stresses (in particular, stress by oxidation), an antithrombotic ability, and an ability of increasing immune strength. Therefore, they can provide not only the effects of protecting blood vessels and improving the fluidity of blood but also an effect of improving tissues in the entire body.

When ascorbic acid or a derivative thereof is contained in the lipid metabolism improving agent of the present invention, the weight ratio of the proanthocyanidins in the pine bark extract and the ascorbic acid or derivative thereof is preferably in the range of 1 : 0.1 to 1 : 50, more preferably 1 : 0.2 to 1 : 20. However, there is no problem even when the amount of ascorbic acid exceeds the above-mentioned ratio.

Among the above-mentioned functional components, mucopolysaccharides, amino sugars, and the like provide an effect of promoting cholesterol excretion as is the case with the pine bark extract. Examples of the vitamins other than ascorbic acid include vitamin A, vitamin B group, vitamin K, and vitamin E. Examples of the water-soluble dietary fibers include indigestible dextrin.

Among the above-mentioned functional components, in particular, components having an ability of suppressing increases in blood glucose level, lipids in blood, and blood pressure can be preferably contained. Also, components providing an effect of preventing a disease or disorder that is closely linked to a cell adhesion factor, such as an antithrombotic effect, an anti-inflammatory effect, and an antitumor effect, can be preferably contained. Examples of such components include sulfur-containing organic compounds, vitamin B group, vitamin K, vitamin E, chitin and derivatives thereof, chitosan and derivatives thereof, mucopolysaccharides, amino sugars, and collagen. Furthermore, hesperidin, quercetine, rutin, and their derivatives having a blood vessel protecting ability and an antioxidation ability can be preferably used.

(III) Other components

Examples of the nutritions that may be contained in the lipid metabolism improving agent of the present invention include, but are not particularly limited to, royal jelly, proteins, minerals, lecithin, chlorella powder, Angelica keiskei powder, and molokheiya powder. Furthermore, it is also possible to add stevia powder, ground green tea powder, lemon powder, honey, maltitol, lactose, sugar solutions, seasonings, and the like so as to control taste.
Examples of the additives that may be contained in the lipid metabolism improving agent of the present invention include excipients, extenders, binders, thickners, emulsifiers, lubricants, humectants, suspending agents, coloring agents, flavors, and food additives.

(IV) Lipid metabolism improving agent

The lipid metabolism improving agent of the present invention contains the above-described pine bark extract and may contain various functional components, nutritions, and additives, if necessary. More specifically, the lipid metabolism improving agent of the present invention can be made into various forms by using these components and subjecting these components to processing that is usually conducted by those skilled in the art.
The lipid metabolism improving agent of the present invention can be processed into various forms. For example, it is possible to add an excipient or the like to a pine bark extract and shape the resultant agent into the form of tablets or pills, or it is possible to make the agent in the form of powder or in other forms without shaping. Examples of other forms of the agent include capsules such as hard capsules and soft capsules, powder, granule, liquid, and paste. Moreover, the agent also can be processed into the forms of tea bags, candy, and the like.
There is no particular limitation on the method for ingesting the lipid metabolism improving agent of the present invention. Depending on the form of the agent or depending on individual preference, the lipid metabolism improving agent may be eaten or drunk as it is, or may be dissolved in water, hot water, milk, or the like and drunk. Alternatively, a liquid containing the components of the agent obtained by percolation may be drunk.
The lipid metabolism improving agent of the present invention contains a pine bark extract in any given ratio. The lipid metabolism improving agent of the present invention can be classified into some types as described below based on the mechanism of action thereof. Generally, when the lipid metabolism improving agent of the present invention is used for food products and pharmaceuticals, it is preferable that the content of the pine bark extract, though it may vary depending on the required mechanism of action (effect), is generally 0.00005 wt% to 50 wt% in terms of the content of proanthocyanidins.
Regarding the intake amount of the lipid metabolism improving agent of the present invention, in order to obtain its effects, the lower limit of the daily intake amount of proanthocyanidins is 0.0005 g, preferably 0.001 g, more preferably 0.02 g, most preferably 0.04 g. Moreover, the upper limit of the daily intake amount of proanthocyanidins is 1.0 g, preferably 0.5 g, more preferably 0.3 g. The value of the daily intake amount may vary depending on the required mechanism of action (effect).
Hereinafter, the types included in the lipid metabolism improving agent of the present invention will be described.

(IV-1) Lipid absorption inhibiting agent

The lipid metabolism improving agent of the present invention has an effect of inhibiting degradation of lipids in the digestion process. Therefore, the lipid metabolism improving agent of the present invention may be used as a lipid absorption inhibiting agent. The lipid absorption inhibiting agent may be processed by the above-described processing method, and may be ingested according to the above-described ingestion method.
The lipid absorption inhibiting agent contains a pine bark extract in any given ratio. For example, when the agent is used for food products or pharmaceuticals, the pine bark extract is contained such that the content of proanthocyanidins is 0.0001 wt% to 50 wt%, preferably 0.001 wt% to 50 wt%, more preferably 0.005 wt% to 20 wt%.
Regarding the intake amount of the lipid absorption inhibiting agent, in order to obtain the above-mentioned effect, the daily intake amount of proanthocyanidins is 0.002 g to 1.0 g, preferably 0.004 g to 0.5 g.

(IV-2) Cholesterol excretion promoting agent

The lipid metabolism improving agent of the present invention has an effect of excreting cholesterol from the body efficiently. Therefore, the lipid metabolism improving agent of the present invention may be used for a cholesterol excretion promoting agent. The cholesterol excretion promoting agent may be processed by the above-described processing method, and may be ingested according to the above-described ingestion method.

Moreover, the cholesterol excretion promoting agent contains a pine bark extract in any given ratio. For example, when the agent is used for food products and pharmaceuticals, the pine bark extract is contained such that the content of proanthocyanidins in each of the food products and pharmaceuticals is 0.001 wt% to 50 wt%, preferably 0.005 wt% to 30 wt%, and more preferably 0.01 wt% to 20 wt%.

Regarding the intake amount of the cholesterol excretion promoting agent, in order to obtain the above-mentioned effect, the daily intake amount of proanthocyanidins is 0.001 g to 1.0 g, preferably 0.02 g to 0.5 g, and more preferably 0.04 g to 0.3 g.

(IV-3) Body fat reducing agent

The lipid metabolism improving agent of the present invention has an effect of inhibiting degradation of lipids in the digestion process and an effect of promoting degradation of absorbed lipids. Therefore, the lipid metabolism improving agent of the present invention may be used as a body fat reducing agent. The body fat reducing agent may be processed by the above-described processing method, and may be ingested according to the above-described ingestion method.

The body fat reducing agent contains a pine bark extract in any given ratio. For example, when the agent is used for food products and pharmaceuticals, the pine bark extract is contained such that the content of proanthocyanidins in each of the food products and pharmaceuticals is 0.00005 wt% to 50 wt%, preferably 0.001 wt% to 50 wt%, and more preferably 0.005 wt% to 20 wt%. The lower limit of the content of the pine bark extract is 0.0001 wt%, preferably 0.001 wt%, more preferably 0.005 wt%, and the upper limit is 50 wt%, preferably 20 wt%.

Regarding the intake amount of the body fat reducing agent, in order to obtain the above-mentioned effects, the daily intake amount of proanthocyanidins is 0.0005 g to 1.0 g, preferably 0.001 g to 0.5 g.

(IV-4) Fat accumulation inhibitory agent

The lipid metabolism improving agent of the present invention has an effect of inhibiting degradation of lipids in the digestion process and other effects. Therefore, the lipid metabolism improving agent of the present invention may be used as a fat accumulation inhibitory agent. The fat accumulation inhibitory agent may be processed in the above-described processing method, and may be ingested according to the above-described ingestion method.

The fat accumulation inhibitory agent contains a pine bark extract in any given ratio. For example, when the agent is used for food products and pharmaceuticals, the pine bark extract is contained such that the content of proanthocyanidins in each of the food products and pharmaceuticals is 0.00005 wt% to 50 wt%, preferably 0.001 wt% to 50 wt%, more preferably 0.005 wt% to 20 wt%. The lower limit of the content of the pine bark extract is 0.0001 wt%, preferably 0.001 wt%, more preferably 0.005 wt%, and the upper limit is 50 wt%, preferably 20 wt%.

Regarding the intake amount of the fat accumulation inhibitory agent, in order to obtain the above-mentioned effects, the daily intake amount of proanthocyanidins is 0.0005 g to 1.0 g, preferably 0.001 g to 0.5 g.

Examples

Hereinafter, the present invention will be described based on examples. However, the present invention is not limited to the examples.

(Example 1) Evaluation of lipid absorption inhibiting ability

A pine bark extract (OPC content was 30 wt%; produced by TOYO SHINYAKU CO., LTD.) obtained by performing an extraction with a mixture of ethanol and water was administered, and the lipid absorption inhibiting ability was evaluated in the following manner.

First, 10 male SD rats (Charles River Laboratories Japan, Inc.) at the age of five weeks were given a standard feed (MF feed, Oriental Yeast Co., Ltd.) for one week for acclimation. Next, the rats were fasted for 16 hours, and then divided into two groups so that the average weights in the two groups were almost equal to each other. Subsequently, blood was collected from the orbit to obtain blood serum. Thereafter, 0.5 ml of cottonseed oil together with 100 mg/kg· body weight of the pine bark extract were orally administered to the rats in one group forcibly using a sonde (this group was taken as the test group), while only 0.5 ml of cottonseed oil were administered to the other group (this group was taken as the control group).

At 1 hour, 2 hours, 4 hours, and 8 hours after the administration, blood was collected from the orbit again, and the neutral fat level in blood was measured using a neutral fat measuring kit (Wako Pure Chemical Industries, Ltd.). The measurement values at the time points after the administration were converted into relative values, wherein the measurement value before the administration was assumed to be 1. Fig. 1 shows average values, and Table 1 shows the average values and standard deviations.

Table 1

| | After 1 hr. | After 2 hr. | After 4 hr. | After 8 hr. |
|---|---|---|---|---|
| Test group | 0.90±0.12 | 1.48±0.45 | 2.10±0.58 | 1.65±0.32 |
| Control group | 1.97±0.45 | 3.17±2.01 | 2.84±0.31 | 3.41±0.47 |
| The values are shown as average value ± standard deviation. | | | | |

The results in Fig. 1 and Table 1 show that in the test group to which the lipid metabolism improving agent of the present invention containing the pine bark extract was given, an increase in neutral fat in blood due to the administration of cottonseed oil could be more suppressed than in the control group to which the agent was not given. Namely, it can be seen that the lipid metabolism improving agent of the present invention has a lipid absorption inhibiting ability and suppresses an increase in neutral fat in blood.

When the above-described test results at 1 hour, 2 hours, 4 hours, and 8 hours after the administration were subjected to two-way analysis of variance, there were significant differences between the test group and the control group at a significance level of 1%. Furthermore, when multiple comparison was performed according to the Tukey method, there were significant differences between the test group and the control group at a significance level of 1%.

(Example 2) Evaluation of cholesterol excretion promoting ability

A pine bark extract (trade name: Flavangenol, produced by TOYO SHINYAKU CO., LTD.) containing 40 wt% of OPCs, 20 wt% of proanthocyanidins having a degree of polymerization of 5 or more, and 10 wt% of catechins was administered, and the cholesterol excretion promoting ability was evaluated in the following manner.

First, 30 SD rats (Charles River Laboratories Japan, Inc.) at the age of four weeks were given a standard feed (MF feed, Oriental Yeast Co., Ltd.) for one week for acclimation. Next, the amount of total cholesterol in blood was measured using a measuring kit (Cholesterol E-Test Wako, Wako Pure Chemical Industries, Ltd.), and the rats were divided into five groups so that the average of the amounts of total cholesterol was almost equal among the groups. Then, 1 wt% of cholesterol, 0.25 wt% of sodium cholate, and 10 wt% of corn oil were added to the standard feed, and furthermore, the pine bark extract was added to the resultant feed in a ratio of 0.02 wt%, 0.2 wt%, or 2.0 wt% to prepare three different types of test feeds. The rats in each of three groups of the five groups were allowed to freely ingest corresponding one type of the three types of test feeds (these groups were taken as the test groups). One group of the remaining two groups was taken as a control and allowed to freely ingest a control feed that was the same as the above-described test feeds except that the pine bark extract was not contained (this group was taken as the control group). Furthermore, the remaining one group was allowed to freely ingest the standard feed in the same manner (this group was taken as the standard group). On the day 24 from the start of the ingestion, feces of each rat were collected. Furthermore, also on the days 25, 26, and 27, feces were collected in the same manner. Lipid components were extracted from the collected feces according to the method of Folch et al. (see Folch J. et al., Journal of Biological Chemistry (J. Biol. chem.), vol. 226, pp. 497-509), and the amount of total cholesterol was measured using the above-mentioned measuring kit. Table 2 shows the results.

Table 2

| | Test group | | | Control group | Standard group |
|---|---|---|---|---|---|
| Pine bark extract content (wt%) | 0.02 | 0.2 | 2 | - | - |
| Cholesterol content (mg/g·feces) | 2.67±0.52 | 3.00±0.56 | 3.09±0.46 | 1.87±0.19 | 0.30±0.04 |
| The values are shown as average value ± standard deviation. | | | | | |

The results in Table 2 show that in the test groups to which the lipid metabolism improving agent of the present invention containing the pine bark extract was given, although the pine bark extract was contained in an amount as small as 0.02 wt%, a cholesterol excretion promoting ability superior to that in the control group to which the agent was not given was exhibited.

When the same test was performed using a grape seed extract (containing 40 wt% of OPCs and 30 wt% of proanthocyanidins having a degree of polymerization of 5 or more) in place of the pine bark extract, only a much lower effect than in the case of the pine bark extract could be obtained.

(Example 3) Evaluation of body fat reducing ability

A pine bark extract (OPC content was 30 wt%; produced by TOYO SHINYAKU CO., LTD.) was administered, and the body fat reducing ability was evaluated in the following manner.

First, 21 SD rats (Charles River Laboratories Japan, Inc.) at the age of four weeks were given a standard feed (MF feed, Oriental Yeast Co., Ltd.) for one week for acclimation. Next, the rats were divided into three groups so that the average weight was almost equal among the groups. Then, 1 wt% of cholesterol, 0.25 wt% of sodium cholate, and 10 wt% of corn oil were added to the standard feed, and furthermore, the pine bark extract was added to the resultant feed in a ratio of 0.02 wt% or 2.0 wt% to prepare two different types of test feeds. The rats in one group of the three groups were allowed to freely ingest one type of the test feeds, and the rats in another group were allowed to freely ingest the other type of the test feeds (these groups were taken as the test groups). The remaining one group was taken as a control and allowed to freely ingest a control feed that was the same as the above-described test feeds except that the pine bark extract was not contained (this group was taken as the control group).

On the day 28 from the start of the ingestion, the weight of each rat was measured. Then, the rats were dissected, and perirenal adipose tissue was excised to measure the amount of fat. The fat weight (%) per body weight was calculated using formula (I) below. Table 3 shows the results.

$$\text{Fat weight (\%) per body weight} = \frac{\text{Fat weight}}{\text{Body weight}} \times 100 \qquad \text{(I)}$$

Table 3

| | Test group | | Control group |
|---|---|---|---|
| Pine bark extract content (wt%) | 0.02 | 2.0 | - |
| Fat weight (wt%) per body weight | 1.26±0.17 | 1.19±0.22 | 1.51±0.19 |
| The values are shown as average value ± standard deviation. | | | |

The results in Table 3 show that in the test groups to which the lipid metabolism improving agent of the present invention containing the pine bark extract was given, the fat weight per body weight was decreased when compared to the control group to which the agent was not given. Namely, it can be seen that the lipid metabolism improving agent of the present invention has a fat reducing ability.

(Example 4) Evaluation of fat accumulation inhibiting ability

A pine bark extract containing 75 wt% of proanthocyanidins and 10 wt% of catechins (OPC content was 40 wt% and the content of proanthocyanidins having a degree of polymerization of 5 or more was 35 wt%; produced by TOYO SHINYAKU CO., LTD.) was administered, and the intrahepatic lipid accumulation inhibiting ability was evaluated in the following manner.

First, 24 SD rats (Charles River Laboratories Japan, Inc.) at the age of four weeks were given a standard feed (MF feed, Oriental Yeast Co., Ltd.) for one week for acclimation. Next, the amount of total cholesterol in blood was measured using a measuring kit (Cholesterol E-Test Wako, Wako Pure Chemical Industries, Ltd.), and the rats were divided into four groups so that the average of the amounts of total cholesterol was almost equal among the groups. Then, 1 wt% of cholesterol, 0.25 wt% of sodium cholate, and 10 wt% of corn oil were added to the standard feed, and furthermore, the pine bark extract was added to the resultant feed in a ratio of 0.2 wt% or 2.0 wt% to prepare two different types of test feeds. The rats in one group of the four groups were allowed to freely ingest one type of the test feeds, and the rats in another group were allowed to freely ingest the other type of the test feeds (these groups were taken as the test groups). One group of the remaining two groups was taken as a control and allowed to freely ingest a control feed that was the same as the above-described test feeds except that the pine bark extract was not contained (this group was taken as the control group). Furthermore, the remaining one group was allowed to freely ingest the standard feed in the same manner (this group was taken as the standard group).

On the day 28 from the start of the ingestion, liver was excised from each rat, and lipid components in the liver tissue were extracted according to the method of Folch et al. (see Folch J. et al., Journal of Biological Chemistry (J. Biol.

chem.), vol. 226, pp. 497-509) to measure the amount of total cholesterol using the above-mentioned measuring kit. Furthermore, triglyceride in the liver also was measured using a measuring kit (Triglyceride G-Test Wako, Wako Pure Chemical Industries, Ltd.). Table 4 shows the results.

Table 4

| | Test group | | Control group | Standard group |
|---|---|---|---|---|
| Pine bark extract content (wt%) | 0.2 | 2.0 | - | - |
| Cholesterol content (mg/g·liver) | 12.6±1.3 | 13.0±1.4 | 20.7±6.4 | 5.3 ±2.9 |
| Triglyceride content (mg/g·liver) | 186.6±17.2 | 148.4±39.5 | 222.8±27.8 | 46.7±4.6 |
| The values are shown as average value ± standard deviation. | | | | |

The results in Table 4 show that in the test groups to which the lipid metabolism improving agent of the present invention containing the pine bark extract was given, cholesterol and triglyceride in the liver were decreased when compared to the control group to which the agent was not given. Namely, the lipid metabolism improving agent of the present invention has an intrahepatic lipid accumulation inhibiting ability and can decrease the amounts of cholesterol and triglyceride in the liver. Therefore, it is believed that when the lipid metabolism improving agent of the present invention is used, prevention of fatty liver and cirrhosis can be expected, and a normal hepatic metabolism can be maintained.

When the same test was conducted using a grape seed extract containing proanthocyanidins in a ratio of 90 wt% in place of the pine bark extract, an intrahepatic lipid accumulation inhibiting ability could be obtained similarly. However, the effect was slightly lower than that in the case of the pine bark extract in which the proanthocyanidin content was 75 wt%.

(Example 5) Evaluation of fat accumulation inhibiting ability and lipid absorption inhibiting ability

A pine bark extract containing proanthocyanidins in a ratio of 60 wt% (OPC content was 30 wt%; produced by TOYO SHINYAKU CO., LTD.) was administered, and the fat accumulation inhibiting ability for visceral fat and subcutaneous fat and the lipid absorption inhibiting ability were evaluated in the following manner.

First, 14 female ICR mice (CLEA Japan, Inc.) at the age of seven weeks were given a standard feed (MF feed, Oriental Yeast Co., Ltd.) for one week for acclimation. Next, the mice were divided into two groups so that the average weights in the two groups were almost equal to each other. Then, the mice in one group were allowed to freely ingest a test feed containing 40 wt% of beef tallow, 9 wt% of granulated sugar, and 5 wt% of the pine bark extract (this group was taken as the test group). The mice in the other group were allowed to freely ingest a control feed that was the same as the test feed except that the pine bark extract was not contained (this group was taken as the control group).

On the day 25 from the start of the ingestion, the weight of each mouse was measured, and the body weight increasing rate was calculated using formula (II) below.

$$\text{Body weight increasing rate (\%)} = \frac{\left(\left(\text{Body weight on day 25 after start of ingestion}\right) - \left(\text{Body weight before ingestion}\right)\right)}{\left(\text{Body weight before ingestion}\right)} \times 100 \qquad \text{(II)}$$

Furthermore, subcutaneous fat was measured using an X-ray CT for experimental animals (trade name: LATheata; produced by ALOKA CO., LTD.). Then, blood was collected from fundus oculi of each mouse, and thereafter the mice were dissected, and retroperitoneal fat and parametrial fat were excised to measure the total weight of these fats (the weight of visceral fat). The collected blood was used to measure the neutral fat with a neutral fat measuring kit (Wako Pure Chemical Industries, Ltd.). Table 5 shows the results.

Table 5

| | Body weight increasing rate (%) | Visceral fat (g) | Subcutaneous fat (g) | Neutral fat (mg/dl) |
|---|---|---|---|---|
| Test group | 30.0±2.4* | 1.36±0.65* | 2.93±1.32* | 74.1±39.5 |

(continued)

| | Body weight increasing rate (%) | Visceral fat (g) | Subcutaneous fat (g) | Neutral fat (mg/dl) |
|---|---|---|---|---|
| Control group | 34.1±2.4 | 2.34±1.01 | 4.56±1.62 | 109.7±62.0 |
| *: A significant difference was present with $p < 0.05$. | | | | |

The results in Table 5 show that in the test group to which the lipid metabolism improving agent of the present invention containing the pine bark extract was given, the amounts of visceral fat and subcutaneous fat were low when compared with the control group to which the agent was not given. Namely, it can be seen that the lipid metabolism improving agent of the present invention has a fat accumulation inhibiting ability for visceral fat and subcutaneous fat. Moreover, a body weight increase was suppressed.

Furthermore, it can be seen that in the test group to which the lipid metabolism improving agent of the present invention containing the pine bark extract was given, neutral fat in blood was decreased when compared with the control group to which the agent was not given. In other words, the lipid metabolism improving agent of the present invention has a lipid absorption inhibiting ability.

(Example 6) Evaluation of fat accumulation inhibiting ability

The same pine bark extract as in Example 5 was administered, and the fat accumulation inhibiting ability was evaluated in the following manner.

(Induction of differentiation into fat cell)

First, mouse 3T3L1 cells suspended in a standard medium (DMEM medium containing 10 vol% inactivated fetal bovine serum) were seeded into a 24-well plate at $3 \times 10^4$ cells per well, and cultured for 48 hours. Next, the standard medium in each of the wells was removed, and 2 ml of a differentiation-inducing medium 1 (DMEM medium containing 0.5 mM 3-isobutyl-1-methylxathine, 1 μM dexamethazone, and 10 vol% inactivated fetal bovine serum) was added to each of the wells. The resultant mixture was allowed to stand for 48 hours for cultivation in order to induce the cultured cells to differentiate into fat cells. Then, the differentiation-inducing medium 1 was removed, and a differentiation-inducing medium 2 (DMEM medium containing 10 μg/ml insulin and 10 vol% inactivated fetal bovine serum) was added, and the resultant mixture was allowed to stand for additional 48 hours for cultivation. Subsequently, the medium was replaced by the standard medium, and the resultant mixture was allowed to stand for 48 hours for cultivation, and thus, differentiation was induced.

(Fat cell accumulation inhibition test)

After the induction of differentiation was completed, the standard medium was removed. Then, four wells each of a test medium (referred to as the "test medium 1") obtained by adding the pine bark extract to the standard medium in a ratio of 0.0001 wt/vol%, a test medium (referred to as the "test medium 2") obtained by adding, in place of the pine bark extract, soy isoflavone (Fuji Oil Co., Ltd.) that is known to have a fat degradation promoting effect to the standard medium, and the standard medium to which nothing was added were prepared. The amount of these media in each of the wells was 2 ml. The resultant three groups were taken as a test medium 1 group, a test medium 2 group, and a standard medium group, respectively, and cultivation was performed for 24 hours. After the cultivation was completed, the media were removed, and the wells were washed three times with PBS(-). Then, a 10 vol% formaldehyde solution was added to the wells at 0.5 ml per well, and the mixtures were allowed to stand at room temperature for one hour to immobilize the cells. Subsequently, the formaldehyde solution was removed, and the wells were washed three times with PBS(-), and thereafter, Oil Red Solution (Wako Pure Chemical Industries, Ltd.) was added to the wells at 0.5 ml per well, and the mixtures were allowed to stand for one hour to stain fats accumulated in the cells. After the staining, the wells were washed three times with PBS(-). Isopropanol was added to the wells at 0.5 ml per well to extract the fats in the cells at room temperature for 30 minutes, and the supernatants were collected. For each of the collected supernatants, absorbance at 490 nm was measured, and average values were obtained. In the measurements, isopropanol was used as a blank, and a fat accumulation inhibiting rate was calculated using the formula (III) below, wherein the fat accumulation inhibiting rate was determined by employing the average value obtained in the standard medium group as 100%. Table 6 shows the results.

$$\text{Fat accumulation inhibiting rate (\%)} = \frac{\left(\left(\begin{array}{c}\text{Average value in}\\\text{standard medium group}\end{array}\right) - \left(\begin{array}{c}\text{Average value in}\\\text{each test medium group}\end{array}\right)\right)}{(\text{Average value in standard medium group})} \times 100 \quad \text{(III)}$$

Table 6

| | Fat accumulation inhibiting rate (%) |
|---|---|
| Test medium 1 (containing pine bark extract) | 57.74 |
| Test medium 2 (containing soy isoflavone) | 2.88 |

The results in Table 6 show that in the test medium 1 group to which the lipid metabolism improving agent of the present invention containing the pine bark extract was added, fat accumulation in the fat cells could be more suppressed than in the test medium 2 group to which the soy isoflavone was added. Namely, it can be seen that the lipid metabolism improving agent of the present invention has a fat accumulation inhibiting ability either by inhibiting fat uptake into cells or by promoting fat degradation in cells.

Industrial Applicability

The lipid metabolism improving agent of the present invention contains a pine bark extract as an active component, and the pine bark extract contains OPCs, proanthocyanidins having a degree of polymerization of 5 or more, and the like. OPCs are easily absorbed into the body and considered to have an ability to promote excretion of cholesterol and degradation of neutral fat in the body to metabolize fats efficiently. On the other hand, proanthocyanidins having a degree of polymerization of 5 or more are considered to inhibit lipid absorption from the deigestive tract by, for example, inhibiting degradation of lipids in the digestion process. Therefore, when the lipid metabolism improving agent of the present invention is utilized for food products and pharmaceuticals, lipid metabolism can be improved.

## Claims

**1.** A lipid metabolism improving agent, comprising a pine bark extract as an active component.

**2.** The lipid metabolism improving agent of claim 1, which is a lipid absorption inhibiting agent.

**3.** The lipid metabolism improving agent of claim 1, which is a cholesterol excretion promoting agent.

**4.** The lipid metabolism improving agent of claim 1, which is a body fat reducing agent.

**5.** The lipid metabolism improving agent of claim 1, which is a fat accumulation inhibitory agent, wherein the fat is visceral fat or subcutaneous fat.

Fig. 1

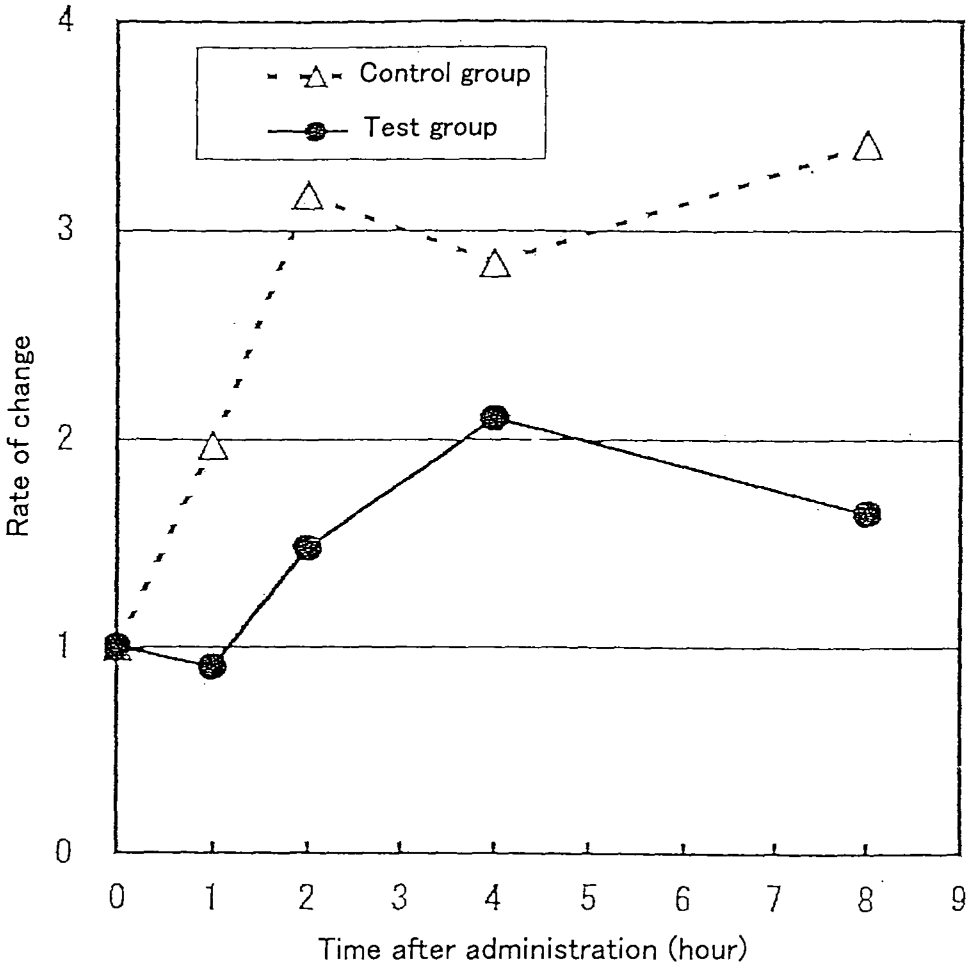

Control group
Test group
Rate of change
0
1
2
3
4
0
1
2
3
4
5
6
7
8
9
Time after administration (hour)

**INTERNATIONAL SEARCH REPORT**

International application No.
PCT/JP2004/016448

A. CLASSIFICATION OF SUBJECT MATTER
Int.Cl$^7$ A61K35/78, 31/352, A61P3/06, A23L1/30

According to International Patent Classification (IPC) or to both national classification and IPC

B. FIELDS SEARCHED

Minimum documentation searched (classification system followed by classification symbols)
Int.Cl$^7$ A61K35/78, 31/352, A61P3/06, A23L1/30

Documentation searched other than minimum documentation to the extent that such documents are included in the fields searched

Electronic data base consulted during the international search (name of data base and, where practicable, search terms used)
CA(STN), BIOSIS(STN), MEDLINE(STN), EMBASE(STN), JICST(JOIS)

C. DOCUMENTS CONSIDERED TO BE RELEVANT

| Category* | Citation of document, with indication, where appropriate, of the relevant passages | Relevant to claim No. |
|---|---|---|
| X | Durackova, Z. et al., Lipid metabolism and erectile function improvement by Pycnogenol, extract from the Pinus pinaster in patients suffering from erctile dysfunztion-a pilot study, Nutrition Research, (September, 2003), Vol.23, No.9, pages 1189 to 1198 | 1-5 |
| X | Sridevi Ddevaraj et al., Supplementation with a Pine Bark Rich in Polyphenols Increases Plasma Antioxidant Capacity and Alters the Plasma Lipoprotein Profile, Lipids, 2002, Vol.37, No.10, pages 931 to 934 | 1-5 |
| X | JP 2003-146898 A (Kabushiki Kaisha Toyo Shin'yaku), 21 May, 2003 (21.05.03), (Family: none) | 1-5 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 26 November, 2004 (26.11.04) | 14 December, 2004 (14.12.04) |

| Name and mailing address of the ISA/ | Authorized officer |
|---|---|
| Japanese Patent Office | |
| Facsimile No. | Telephone No. |

Form PCT/ISA/210 (second sheet) (January 2004)

**INTERNATIONAL SEARCH REPORT**

International application No.
PCT/JP2004/016448

C (Continuation). DOCUMENTS CONSIDERED TO BE RELEVANT

| Category* | Citation of document, with indication, where appropriate, of the relevant passages | Relevant to claim No. |
|---|---|---|
| Y | JP 2001-106634 A (Nobutaka SUZUKI), 17 April, 2001 (17.04.01), (Family: none) | 1-5 |
| Y | JP 9-291039 A (Suntory Ltd.), 11 November, 1997 (11.11.97), & WO 97/23210 A1 & EP 815857 A1 & US 629410 B1 | 1-5 |
| Y | JP 8-259557 A (Lotte Co., Ltd.), 08 October, 1996 (08.10.96), & US 5629338 A | 1-5 |
| Y | JP 2002-538802 A (LABORATOIRES ARKOPHARMA), 19 November, 2002 (19.11.02), & FR 2790645 A & WO 00/54610 A1 & AU 3169500 A & EP 1161157 A & BR 8926 A & CN 1343100 A & US 6638545 B1 | 1-5 |

Form PCT/ISA/210 (continuation of second sheet) (January 2004)